# EUROPÄISCHE PATENTSCHRIFT

(11) **EP 0 780 360 B1**
(45) Veröffentlichungstag und Bekanntmachung des Hinweises auf die Patenterteilung: **26.04.2000**
(21) Anmeldenummer: 96120076.3
(22) Anmeldetag: 13.12.1996
(51) Int. Cl.: C07C 67/60, C07C 67/08, C07C 69/54

(54) **Verfahren zur Veresterung von (Meth)acrylsäure mit einem Alkanol**
Esterification process of (meth)acrylic acid with an alcanol
Procédé d'esterification d'acide (méth)acrylique avec un alcanol

(30) Priorität: 19.12.1995 DE 19547459
(43) Veröffentlichungstag der Anmeldung: 25.06.1997
(73) Patentinhaber: BASF AKTIENGESELLSCHAFT, 67056 Ludwigshafen (DE)
(72) Erfinder: Nestler, Gerhard, Dr., 67061 Ludwigshafen (DE); Aichinger, Heinrich, Dr., 68199 Mannheim (DE); Herbst, Holger, Dr., 67227 Frankenthal (DE); McGlone, Nicole E., Friendswood, TX 77546 (US); Darlington, Jerry W., Marengo III 60152 (US); Donath, Calvin F., Lake Jackson, TX 77566 (US); Heimann, John C. VERSTORBEN, West Columbia, TX 77486 (US)
(74) Vertreter: Isenbruck, Günter, Dr.

(56) Entgegenhaltungen:
- DE-A- 2 339 519

## Beschreibung

Die Erfindung betrifft ein Verfahren zum Verestern von (Meth)acrylsäure mit einem Alkanol in Gegenwart eines Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht. Der Begriff (Meth)acrylsäure bezeichnet dabei in bekannter Weise Acryl- oder Methacrylsäure.

Die Herstellung von Alkylestern der (Meth)acrylsäure erfolgt üblicherweise durch Verestern von (Meth)acrylsäure mit Alkanolen bei erhöhter Temperatur in flüssiger Phase mit oder ohne Lösungsmittel und in Gegenwart von Säure als Katalysator (DE-A 23 39 519). Nachteilig an dieser Herstellungsweise ist, daß sich unter den vorgenannten Veresterungsbedingungen als Nebenreaktionen noch nicht umgesetzter Ausgangsalkohol unter Ausbildung einer Verbindung der nachfolgend angeführten allgemeinen Formel I sowie noch nicht umgesetzte (Meth)acrylsäure unter Ausbildung einer Verbindung der allgemeinen Formel II an die Doppelbindung von bereits gebildetem (Meth)acrylsäurealkylester addiert (Michael-Addition). Auch eine Mehrfachaddition ist möglich. Ferner können gemischte Typen auftreten. Diese Addukte (Alkoxyester und Acyloxyester) nennt man kurz Oxyester.

RO―(CH₂―CHR'―CO₂)ₓ ―R (I)

CH₂=CR'―CO₂―(CH₂―CHR'―CO₂)_{y}―R (II)

mit
x,y = 1- 5
R = Alkyl
R' = H oder CH₃
Wenn R' = H ist, handelt es sich um eine Veresterung der Acrylsäure, wenn R' = CH₃ ist, handelt es sich um eine Veresterung der Methacrylsäure.

Bei der Herstellung von Estern der Acrylsäure ist das Problem der Oxyesterbildung besonders akut, wobei die hauptsächlich gebildeten Oxyester der Alkoxypropionsäureester und der Acyloxypropionsäureester mit x,y = 1 sind. Bei der Herstellung von Estern der Methacrylsäure erfolgt die Oxyesterbildung in geringerem Maße. Die Entstehung von Oxyestern ist in der DE-A 23 39 529 beschrieben. Aus dieser geht hervor, daß die Bildung von Oxyestern im wesentlichen unabhängig von den speziellen Veresterungsbedingungen erfolgt. Von ganz besonderer Bedeutung ist die Oxyesterbildung bei der Herstellung von Acrylaten der C₁- bis C₈-Alkanole, insbesondere der C₄- bis C₈-Alkanole, ganz besonders bei der Herstellung von n-Butylacrylat und 2-Ethylhexylacrylat.

Charakteristisch für die Oxyester ist, daß ihr Siedepunkt oberhalb der Siedepunkte von Ausgangssäure, Ausgangsalkohol, gebildetem Zielester sowie gegebenenfalls mitverwendetem organischem Lösungsmittel liegt.

Die Aufarbeitung eines beliebigen Veresterungs-Reaktionsgemisches erfolgt normalerweise so, daß nicht umgesetzte Ausgangsverbindungen sowie der Zielester vom Reaktionsgemisch destillativ abgetrennt werden, wobei der zur Veresterung verwendete Säurekatalysator gegebenenfalls vorher durch Extraktion mittels Wasser und/oder wäßriger Lauge abgetrennt wird (vgl. z.B. Ullmann's Encyclopedia of Industrial Chemistry, Vol. A1, 5th Ed., VCH, S. 167ff). Das im Rahmen einer solchen destillativen Aufarbeitung verbleibende Sumpfprodukt enthält die Oxyester, die einen beträchtlichen Ausbeuteverlust bedingen.

Es sind daher weitere verschiedenartige Verfahren untersucht worden, um die durch das Auftreten der Oxyester entstehenden Probleme zu lösen. So beschreibt die JP-A-82/62229 die alkalische Verseifung des hochsiedenden Veresterungsrückstandes. Man erhält auf diese Weise einen Teil des eingesetzten Alkohols und Acrylsäure und β-Hydroxypropionsäure bzw. deren Salze zurück. Eine einfache und wirtschaftliche Rückführung der Produkte in die Veresterungsreaktion ist daher nicht möglich. Die JP-AS-72/15936 beschreibt die Gewinnung von Acrylestern durch Umsetzung von β-Alkoxypropionsäureestern mit Acrylsäure in Gegenwart von starken Säuren (Umesterung). Als Nebenprodukt fallen dabei jedoch äquimolare Mengen an β-Alkoxypropionsäure an, die nicht in die Veresterungsreaktion zurückgeführt werden können und daher Abfall darstellen. Die JP-A-93/25086 beschreibt die Spaltung des Michael-Additionsproduktes β-Butoxypropionsäurebutylester (s. Formel I, x = 1, R = Butyl) bei erhöhter Temperatur und in Gegenwart von Schwefelsäure und einem Überschuß an Wasser. Der Umsatz beträgt jedoch lediglich ca. 30 %. Schließlich beschreibt JP-A-94/65149 die Spaltung der Michael-Additionsprodukte I und II (s.o. x = y = 1) in Gegenwart von Titanalkoholaten. Hier ist der Umsatz ebenfalls gering (< 60%) und es sind hohe Titanatmengen notwendig. Daher ist dieses Verfahren unwirtschaftlich und wegen der großen zu entsorgenden Titanatmengen umweltbelastend.

In der GB 923 595 ist die Gewinnung von Monomeren aus dem Rückstand der Veresterung von Acrylsäure mit Alkanolen in Abwesenheit von molekularem Sauerstoff beschrieben. Empfohlen wird u.a. die Entfernung von allen flüchtigen Monomeren vor der Spaltung, die Spaltung in Gegenwart von Schwefelsäure und die Entfernung der Spaltprodukte mit Hilfe eines Inertgasstromes. Entsprechend den Ausführungsbeispielen wird die Spaltung immer bei min. 300 °C durchgeführt. Als Rückstand entsteht Koks (17-40%). Dieser muß "bergmännisch" aus dem Reaktor entfernt werden. Daher ist dieses Verfahren weder wirtschaftlich noch im technischen Maßstab durchführbar. Ein weiterer Nachteil ist der notwendige Sauerstoffausschluß.

Die CN-A 1,063,678 beschreibt die Spaltung des im Veresterungsrückstand enthaltenen Alkoxypropionsäureesters in Gegenwart von Schwefelsäure in einer Kaskade, wobei Temperatur und Katalysatorkonzentration (0,8 - 1,5 %) in jedem Reaktor unterschiedlich sind. Gekoppelt mit der Spaltung ist eine Destillation zur Trennung von Alkanol und Acrylat. Das Verfahren ist sehr umständlich und erreicht keine hohen Umsätze.

Schließlich beschreibt die CN-A 1,058,390 die Spaltung von Alkoxypropionsäureestern in Gegenwart von Schwefelsäure etc. in Alkanole und Acrylsäureester. Es wird dabei schrittweise vorgegangen. Zuerst wird die Spaltung unter Rückfluß durchgeführt und anschließend werden die Reaktionsprodukte abdestilliert. Die Spaltung der acrylsäurehaltigen Esterrückstände der Ethyl/Methylacrylatherstellung (Ethylethoxypropionat, Methylmethoxypropionat) wird in Gegenwart von Ethanol bzw. Methanol durchgeführt. Auch hier ist das Verfahren kompliziert und erreicht keine hohen Umsätze.

Der Erfindung liegt die Aufgabe zugrunde, die Rückspaltung der in diesem Sumpfprodukt enthaltenen Oxyester und die Weiterverwendung der dabei anfallenden Ausgangssäure, Ausgangsalkohol und Zielester im Rahmen der Veresterung ohne die Nachteile der Verfahren nach dem Stand der Technik durchzuführen.

Diese Aufgabe wird erfindungsgemäß gelöst durch ein Verfahren zum Verestern von (Meth)acrylsäure mit einem Alkanol in Gegenwart eines Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht, dadurch gekennzeichnet, daß das Sumpfprodukt abgetrennt wird und daß entweder (a) das Sumpfprodukt unmittelbar mit (Meth)acrylsäure versetzt wird und danach die im Sumpfprodukt enthaltenenen Oxyester in Anwesenheit von von (Meth)acrylsäure verschiedenen Säurekatalysatoren unter Einwirkung erhöhter Temperatur gespalten werden oder (b) die Oxyester zunächst aus dem Sumpfprodukt destillativ abgetrennt werden, das Destillat mit (Meth)acrylsäure versetzt und dann in Anwesenheit von von (Meth)acrylsäure verschiedenen Säurekatalysatoren unter Einwirkung erhöhter Temperatur gespalten wird, wobei die Spaltung drucklos oder bei reduziertem Druck (< 1 atm) durchgeführt wird. Das Alkanol ist vorzugsweise n-Butanol oder 2-Ethylhexanol. In der Regel werden, bezogen auf die Menge des Sumpfproduktes in (a) oder des Destillats in (b), 5 bis 50 Gew.-%, vorzugsweise 10 bis 40 Gew.-% (Meth)acrylsäure zugesetzt. Normalerweise erfolgt der Zusatz der (Meth)acrylsäure in an sich bekannter, mittels Polymerisationsinhibitoren stabilisierter Form. Gemäß einer vorteilhaften Ausbildung der Erfindung wird das Verfahren der Spaltung im Beisein von molekularem Sauerstoff durchgeführt.

Im Unterschied zu einer Spaltung ohne Zusatz von (Meth)acrylsäure, bedingt das erfindungsgemäße Verfahren eine geringere Nebenproduktbildung (Dialkylether, Olefine).

Gemäß einer vorteilhaften Weiterbildung der Erfindung werden dem Sumpfprodukt zusätzlich zu dem ggf. bereits enthaltenen sauren von (Meth)acrylsäure verschiedenen Veresterungskatalysator weitere Säuren aus der Gruppe umfassend Mineralsäuren, wie Schwefelsäure oder Phosphorsäure, und Alkyl- oder Arylsulfonsäuren, beispielsweise Methansulfonsäure oder p-Toluolsulfonsäure, zugesetzt. Dabei kann die dann insgesamt enthaltene von (Meth)acrylsäure verschiedene Menge an Säuren 1 bis 20 Gew.-%, vorzugsweise 5 bis 15 Gew.-%, bezogen auf die Menge des Sumpfprodukts in (a) oder des Destillats in (b) betragen. Entsprechende katalytische Säuregehalte erweisen sich für die Rückspaltung im Falle einer der Rückspaltung vorausgehenden destillativen Abtrennung der Oxyester als günstig. Besonders günstig ist es, wenn als Schleppmittel für die Spaltprodukte ein Strippgas, das vorzugsweise molekularen Sauerstoff enthält, durch das Sumpfprodukt in (a) bzw. durch das aus diesem gewonnene Destillat in (b) geführt wird. Zweckmäßigerweise werden als Strippgas Luft oder Gemische von Luft mit Inertgas (z.B. Stickstoff) verwendet.

Die Vorteile des erfindungsgemäßen Verfahrens sind vor allem darin zu sehen, daß bei diesem die Spaltung schneller abläuft, weniger Nebenprodukte wie Ether oder Olefine entstehen und daß weniger an saurem Spaltkatalysator verbraucht wird. Somit treten u.a. geringere Verluste an Einsatzstoffen, vor allem an Alkoholen auf als mit bekannten Verfahren. Außerdem sind hohe Spaltumsätze erzielbar. Die direkte Rückführung des Spaltgemisches verursacht keine Verschlechterung der (Meth)acrylesterreinheit und führt zu einem geringen Ethergehalt. Es ist daher auch keine aufwendige Abtrennung des Ethers vom leicht polymerisierbarem (Meth)acrylester notwendig.

Bei der destillativen Abtrennung der Oxyester vom Sumpfprodukt richten sich die Destillationsbedingungen nach der Art der bei der Veresterung eingesetzten Alkoholkomponente. In der Regel sind eine Temperatur von 100 bis 300 °C und ein Druck von 1 bis 50 mbar vorgesehen. Für das Destillationsverfahren eignet sich jede herkömmliche Destillationsapparatur. Da nur eine einfache Trennaufgabe zu lösen ist, genügt in der Regel ein einfacher Spritzschutz, d.h. eine Kolonne ist normalerweise nicht erforderlich.

Für die erfindungsgemäße Aufarbeitung der bei der Veresterung im Sumpfprodukt anfallenden Oxyester bzw. des vom Veresterungssumpf abgetrennten Oxyesterdestillats kann ein einfacher beheizbarer Rührreaktor mit Doppelwandheizung oder Heizschlange oder auch ein Zwangsumlaufverdampfer, beispielsweise ein Fallfilmverdampfer oder Flashverdampfer, gekoppelt mit einem Verweilzeitbehälter, verwendet werden. Zur besseren Trennung der Spaltprodukte vom Sumpfprodukt bzw. Oxyesterdestillat ist ggf. eine auf die Spaltapparatur aufgesetzte Rektifikationsvorrichtung z.B. eine Füllkörper-, Packungs- oder Bodenkolonne zweckmäßig. Diese Rektifikationsvorrichtung wird in der Regel mit Polymerisationsinhibitoren (z.B. Phenothiazin, Hydrochinonmonomethylether etc.) stabilisiert betrieben.

Die Bedingungen für die Durchführung des erfindungsgemäßen Verfahrens der Spaltung der bei der Veresterung im Sumpfprodukt anfallenden bzw. vom Sumpfprodukt abgetrennten Oxyester sind folgende:
- Katalysator:: wenigstens eine Säure aus der Gruppe umfassend Mineralsäuren, wie z.B. Schwefelsäure und Phosphorsäure, sowie von (Meth)acrylsäure verschiedene organische Säuren wie z.B. Alkyl- oder Arylsulfonsäuren, beispielsweise Methansulfonsäure oder p-Toluolsulfonsäure
- Katalysatormenge:: 1 - 20 Gew.-%, vorzugsweise 5 - 15 Gew.-% bezogen auf die Menge des Sumpfprodukts in (a) bzw. auf die Menge des vom Sumpfprodukt abgetrennten Oxyesterdestillats in (b)
- (Meth)acrylsäuremenge:: 5 - 50, vorzugsweise 10 - 40 Gew.-%, bezogen auf die Menge des Sumpfprodukts in (a) bzw. auf die Menge des vom Sumpfprodukt abgetrennten Oxyesterdestillats in (b)
- Temperatur:: 150 - 250 °C, vorzugsweise 180 - 230 °C
- Druck:: drucklos bzw. reduzierter Druck (< 1 atm)
(so daß die Spaltprodukte unmittelbar abdampfen)
- ggf. Strippgas: Menge: 1 - 100 l/h 1
- Reaktionszeit:: 1 - 10 Std.
- Umsatz:: ≥ 90%

Die Reaktionsführung läuft z.B. in der Weise, daß das zu spaltende Sumpfprodukt kontinuierlich aus der destillativen Aufarbeitung des Veresterungsgemisches ausgeschleust und mit dem Spaltkatalysator dem Spaltreaktor zugeführt wird. Die Reaktionsführung kann aber auch diskontinuierlich, d.h. batchweise, durchgeführt werden. Auch eine halbkontinuierliche Reaktionsführung ist möglich, bei der das zu spaltende Produkt kontinuierlich dem Spaltreaktor, der den Spaltkatalysator enthält, zugeführt wird und das Sumpfprodukt erst nach Beendigung der Spaltung aus dem Spaltreaktor batchweise entfernt wird. Die Spaltprodukte werden kontinuierlich destillativ abgetrennt.

Die Anwendbarkeit des beschriebenen Spaltverfahrens ist nicht auf eine spezielle Natur des Veresterungsprozesses, als dessen Nebenprodukte die Oxyester, also die Additionsverbindungen I und II, anfallen, beschränkt. In der Regel werden die Ester nach den üblichen Verfahren hergestellt (s. Ullmann's Encyclopedia of Industrial Chemistry, Vol. A1, 5th Ed., VCH, S. 167ff).

Ein typisches Beispiel für die Bedingungen, unter denen die der Spaltung der Oxyester vorausgehende Veresterung stattfinden kann, lassen sich kurz wie folgt darstellen:
- Alkohol:: (Meth)acrylsäure 1 : 0,7 - 1,2 (molar)
- Katalysator:: Schwefelsäure oder Sulfonsäuren
- Katalysatormenge:: 0,1 - 10 Gew.-% (vorzugsweise 0,5 - 5 Gew.-%) bzgl. Einsatzstoffe
- Stabilisierung:: 200 - 2000 ppm Phenothiazin (bezogen auf das Gewicht der Einsatzstoffe)
- Reaktionstemperatur:: 80 - 160 °C, vorzugsweise 90 - 130 °C
- Reaktionszeit:: 1 - 10 Std., vorzugsweise 1 - 6 Std.

Gegebenenfalls wird ein Schleppmittel (z.B. Cyclohexan oder Toluol) zur Entfernung des Veresterungswassers eingesetzt. Die Veresterung kann drucklos, oder mit Unterdruck sowohl kontinuierlich als auch diskontinuierlich durchgeführt werden.

Bei der säurekatalysierten Veresterung von Acrylsäure mit Alkanolen hat das nach der Abtrennung des sauren Veresterungskatalysators, der nicht umgesetzten Ausgangsstoffe und des Acrylesters resultierende Sumpfprodukt in der Regel folgende Zusammensetzung:
1 - 20 Gew.-% Acrylester
50 - 80 Gew.-% Alkoxypropionate (s. Formel I)
5 - 30 Gew.-% Acyloxypropionate (s. Formel II)
Rest: hauptsächlich Stabilisatoren (Phenothiazin) und Polymere

Weitere Einzelheiten und Vorteile des erfindungsgemäßen Verfahrens können dem im folgenden beschriebenen Ausführungsbeispiel entnommen werden.

Zunächst soll anhand eines Vergleichsbeispiels ein mit einem nicht erfindungsgemäßen Verfahren erzieltes Ergebnis beschrieben werden.

### Vergleichsbeispiel

Ein aus Glas bestehender Umlaufreaktor (Volumen: 1 1), beheizt mit einer Heizkerze, wurde mit 500 g eines Oxyesterdestillats, gewonnen von vom sauren Veresterungskatalysator befreiten Veresterungsrückstand der n-Butylacrylatherstellung, und 40 g p-Toluolsulfonsäure gefüllt. Das Oxyesterdestillat enthielt
11,0 Gew.-% Butylacrylat,
64,8 Gew.-% Butoxyester I (R = C₄H₉)
20,5 Gew.-% Acyloxyester II (R = C₄H₉).

Die Spalttemperatur betrug 195 °C und der Arbeitsdruck lag bei 1 atm.

Standgeregelt wurde dem Spaltreaktor während der Spaltung kontinuierlich der zu spaltende Veresterungsrückstand zugeführt.

Die Spaltprodukte wurden dampfförmig abgeführt und am Kopf der auf dem Spaltreaktor aufgesetzten Kolonne (50 cm x 2,8 cm, leer) kondensiert. Innerhalb von 119,5 Stunden wurden 7401 g Gemisch (62 g/h) der Spaltung zugeführt und 7080 g Spaltprodukte kondensiert.

Entsprechend der gaschromatographischen Analyse enthielt das Kondensat:
72,0 Gew.-% Butylacrylat
13,9 Gew.-% Butanol
4,8 Gew.-% Acrylsäure
1,4 Gew.-% Dibutylether
6,6 Gew.-% Butene
0,2 Gew.-% Butoxypropionsäurebutylester
Umsatz: 96 Gew.-% bezogen auf Oxyester

Der Spaltsumpf war bei 25 °C noch gut handhabbar (pumpfähig) und enthielt keine Feststoffe.

### Beispiel des erfindungsgemäßen Verfahrens

Ein aus Glas bestehender Umlaufreaktor (Volumen 1 1), beheizt mit einer Heizkerze, wurde mit 500 g des Oxyesterdestillats aus dem Vergleichsbeispiel gefüllt, wobei zusätzlich 40 g p-Toluolsulfonsäure und 150 g Acrylsäure (mit 300 ppm Phenothiazin stabilisiert) zugesetzt wurden.

Die Spalttemperatur betrug 195 °C der Arbeitsdruck betrug 1 atm.

Standgeregelt wurde dem Spaltreaktor das zu spaltende Oxyesterdestillat und der entsprechender Acrylsäurezusatz (30 Gew.-%) kontinuierlich zugeführt. Die Spaltprodukte wurden am Kopf der auf dem Reaktor aufgesetzten Kolonne (50 cm x 2,8 cm, leer) kondensiert.

Innerhalb von 118 Stunden wurde 12323 g Oxyesterdestillat (104 g/h) und 3697 g stabilisierte Acrylsäure der Spaltung zugeführt und 15529 g Produktgemisch kondensiert. Entsprechend der gaschromatographischen Analyse enthielt das Kondensat:
67,3 Gew.-% Butylacrylat
5,3 Gew.-% Butanol
21,2 Gew.-% Acrylsäure
0,4 Gew.-% Dibutylether
2,3 Gew.-% Butene
Umsatz: 97 Gew.-%, bezogen auf Oxyester.

Dem vorstehenden Beispiel des erfindungsgemäßen Verfahren ist zu entnehmen, daß mit diesem Verfahren höhere Umsätze erzielbar sind und daß geringere Verluste an Einsatzmaterial auftreten als bei bekannten Verfahren.

## Patentansprüche

1. Verfahren zum Verestern von (Meth)acrylsäure mit einem Alkanol in Gegenwart eines Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht, **dadurch gekennzeichnet, daß** das Sumpfprodukt abgetrennt wird und daß entweder
(a) das Sumpfprodukt unmittelbar mit (Meth)acrylsäure versetzt wird und danach die im Sumpfprodukt enthaltenen Oxyester in Anwesenheit von von (Meth)acrylsäure verschiedenen Säurekatalysatoren unter Einwirkung erhöhter Temperatur gespalten werden oder
(b) die Oxyester zunächst aus dem Sumpfprodukt destillativ abgetrennt werden, das Destillat mit (Meth)acrylsäure versetzt und dann in Anwesenheit von von (Meth)acrylsäure verschiedenen Säurekatalysatoren unter Einwirkung erhöhter Temperatur gespalten wird, wobei die Spaltung drucklos oder bei reduziertem Druck (< 1 atm) durchgeführt wird.

2. Verfahren nach Anspruch 1, dadurch gekennzeichnet, daß die zugesetzte Menge an (Meth)acrylsäure 5 bis 50 Gew.-%, vorzugsweise 10 bis 40 Gew.-%, bezogen auf die Menge des Sumpfprodukts in (a) oder des Destillats in (b), beträgt.

3. Verfahren nach Anspruch 1 oder 2, dadurch gekennzeichnet, daß das Verfahren der Spaltung im Beisein von molekularem Sauerstoff durchgeführt wird.

4. Verfahren nach einem der Ansprüche 1 bis 3, dadurch gekennzeichnet, daß die Spaltung bei Temperaturen von 150 bis 250°C, vorzugsweise bei 180 bis 230°C durchgeführt wird.

5. Verfahren nach einem der Ansprüche 1 bis 4, dadurch gekennzeichnet, daß als Säurekatalysator eine Säure aus der Gruppe umfassend Mineralsäuren, wie Schwefelsäure oder Phosphorsäure, und von (Meth)acrylsäure verschiedene organische Säuren, wie Alkyl- oder Arylsulfonsäuren, z.B. Methansulfonsäure oder p-Toluolsulfonsäure, zugesetzt wird.

6. Verfahren nach Anspruch 5, dadurch gekennzeichnet, daß die Menge an zugesetztem Säurekatalysator 1 bis 20 Gew.-%, vorzugsweise 5 bis 15 Gew.-%, bezogen auf die Menge des Sumpfproduktes in (a) oder des Destillates in (b), beträgt.

7. Verfahren nach einem der Ansprüche 1 bis 6, dadurch gekennzeichnet, daß zur Entfernung der anfallenden Spaltprodukte ein Strippgas durch das Sumpfprodukt in (a) oder das Destillat in (b) geführt wird.

8. Verfahren nach Anspruch 7, dadurch gekennzeichnet, daß als Strippgas ein Sauerstoff enthaltendes Gas verwendet wird.

9. Verfahren nach einem der Ansprüche 1 bis 8, dadurch gekennzeichnet, daß die anfallenden Spaltprodukte unmittelbar in die Veresterung rückgeführt werden.

## Claims

1. A process for the esterification of (meth)acrylic acid with an alkanol in the presence of an esterification catalyst, in which unreacted starting compounds and the (meth)acrylic ester formed are removed by distillation, and a bottom product containing oxy esters results, which comprises removing the bottom product and either
(a) immediately adding (meth)acrylic acid to the bottom product and then cleaving the oxy esters ,present in the bottom product in the presence of acid catalysts which are different from (meth)acrylic acid with exposure to elevated temperature or
(b) initially removing the oxy esters from the bottom product by distillation, adding (meth)acrylic acid to the distillate, and then cleaving in the presence of acid catalysts which are different from (meth)acrylic acid with exposure to elevated temperature, the cleavage being carried out under atmospheric pressure or under reduced pressure (< 1 atm).

2. A process as claimed in claim 1, wherein the amount of (meth)acrylic is from 5 to 50% by weight, preferably 10 to 40% by weight, based on the amount of the bottom product in (a) or of the distillate in (b).

3. A process as claimed in claim 1 or 2, wherein the process of cleavage is carried out in the presence of molecular oxygen.

4. A process as claimed in any of claims 1 to 3, wherein the cleavage is carried out at from 150 to 250°C, preferably at 180 to 230°C.

5. A process as claimed in any of claims 1 to 4, wherein the acid catalyst added is an acid from the group consisting of mineral acids such as sulfuric acid or phosphoric acid, and organic acids which are different from (meth)acrylic acid, such as alkyl- or arylsulfonic acids, eg. methanesulfonic acid or p-toluenesulfonic acid.

6. A process as claimed in claim 5, wherein the amount of added acid catalyst is from 1 to 20% by weight, preferably 5 to 15% by weight, based on the amount of the bottom product in (a) or of the distillate in (b).

7. A process as claimed in any of claims 1 to 6, wherein a stripping gas is passed through the bottom product in (a) or the distillate in (b) in order to remove the resulting cleavage products.

8. A process as claimed in claim 7, wherein an oxygen-containing gas is used as stripping gas.

9. A process as claimed in any of claims 1 to 8, wherein the resulting cleavage products are recycled directly to the esterification.

## Revendications

1. Procédé d'estérification de l'acide (méth)acrylique avec un alcanol en présence d'un catalyseur d'estérification, dans lequel les composés de départ n'ayant pas réagi et l'ester d'acide (méth)acrylique formé sont séparés par distillation et un produit de fond contenant un oxyester est formé, caractérisé en ce que le produit de fond est séparé et que
(a) soit le produit de fond est mélangé directement avec l'acide (méth)acrylique et ensuite les oxyesters contenus dans le produit de fond sont dissociés sous l'effet d'une température élevée en présence de catalyseurs acides différents de l'acide (méth)acrylique,
(b) soit les oxyesters sont d'abord séparés par distillation d'avec le produit de fond, le distillat est mélangé à l'acide (méth)acrylique puis dissocié sous l'effet d'une température élevée, en présence de catalyseurs acides différents de l'acide (méth)acrylique,
la dissociation étant effectuée sans pression ou sous pression réduite (< 1 atm).

2. Procédé selon la revendication 1, caractérisé en ce que la quantité ajoutée d'acide (méth)acrylique est de 5 à 50% en poids, de préférence de 10 à 40% en poids, par rapport à la quantité du produit de fond dans (a) ou du distillat dans (b).

3. Procédé selon la revendication 1 ou 2, caractérisé en ce que le procédé de dissociation est réalisé en présence d'oxygène moléculaire.

4. Procédé selon l'une quelconque des revendications 1 à 3, caractérisé en ce que la dissociation est réalisée à des températures de 150 à 250°C, de préférence de 180 à 230°C.

5. Procédé selon l'une quelconque des revendications 1 à 4, caractérisé en ce qu'un acide choisi dans le groupe formé par les acides minéraux, tels que l'acide sulfurique ou l'acide phosphorique, et les acides organiques différents de l'acide (méth)acrylique, tels que les acides alkyl- ou arylsulfoniques, par exemple l'acide méthanesulfonique ou l'acide p-toluènesulfonique, est ajouté en tant que catalyseur acide.

6. Procédé selon la revendication 5, caractérisé en ce que la quantité ajoutée de catalyseur acide est de 1 à 20% en poids, de préférence 5 à 15% en poids, par rapport à la quantité du produit de fond dans (a) ou du distillat dans (b).

7. Procédé selon l'une quelconque des revendications 1 à 6, caractérisé en ce que, pour évacuer les produits obtenus lors de la dissociation, un gaz d'extraction est introduit à travers le produit de fond dans (a), ou le distillat dans (b).

8. Procédé selon la revendication 7, caractérisé en ce qu'on utilise un gaz contenant de l'oxygène en tant que gaz d'extraction.

9. Procédé selon l'une quelconque des revendications 1 à 8, caractérisé en ce que les produits obtenus lors de la dissociation sont directement renvoyés dans l'estérification.
